(19) Europäisches Patentamt
European Patent Office
Office européen des brevets

(11) Numéro de publication: **0 050 084**
**B1**

# (12) FASCICULE DE BREVET EUROPEEN

(45) Date de publication du fascicule du brevet: **21.11.84**

(51) Int. Cl.³: **C 07 C 51/56**, C 07 C 53/12

(21) Numéro de dépôt: **81420140.6**

(22) Date de dépôt: **28.09.81**

## (54) Procédé de préparation d'anhydrides d'acides carboxyliques par carbonylation.

(30) Priorité: **09.10.80 FR 8022088**

(43) Date de publication de la demande:
**21.04.82 Bulletin 82/16**

(45) Mention de la délivrance du brevet:
**21.11.84 Bulletin 84/47**

(84) Etats contractants désignés:
**AT BE CH DE FR GB IT LI LU NL SE**

(56) Documents cités:
**DE - A - 930 327**
**DE - A - 965 320**
**FR - A - 2 289 480**
**FR - A - 2 336 367**
**FR - A - 2 455 021**
**GB - A - 1 293 193**

(73) Titulaire: **RHONE-POULENC CHIMIE DE BASE, 25, quai Paul Doumer, F-92408 Courbevoie (FR)**

(72) Inventeur: **Gauthier-Lafaye, Jean, 21, rue Duguesclin, F-69006 Lyon (FR)**
Inventeur: **Perron, Robert, La Pécolière, F-69390 Charly (FR)**

(74) Mandataire: **Varniere-Grange, Monique et al, RHONE-POULENC RECHERCHES Service Brevets Chimie et Polymères Centre de Recherches de Saint-Fons B.P. 62, F-69192 St-Fons Cédex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 050 084 B1

BUNDESDRUCKEREI BERLIN

**Description**

La présente invention a pour objet un procédé de préparation d'anhydrides d'acides carboxyliques, et plus particulièrement de l'anhydride acétique, par carbonylation d'esters carboxyliques.

Il est bien connu que l'anhydride acétique peut être produit par carbonylation de l'acétate de méthyle, dans des conditions extrêmement sévères de pression en présence de complexes du nickel de formule

$$[A_4M]_2NiX_4$$

dans laquelle X représente un atome de brome ou d'iode, M un atome de phosphore ou d'azote, A étant, par exemple, un radical alkyle inférieur (Cf. brevet des ETATS UNIS D'AMERIQUE n° 2 729 651). Ces complexes, obtenus par réaction d'halogénures de nickel et d'halogénures de phosphonium ou d'ammonium quaternaire sont le plus souvent engagés à la réaction en cause sous forme d'une solution dans un N-alkyl lactame. Toutefois l'efficacité de ce type de procédé est faible malgré les hautes pressions mises en œuvre.

Par ailleurs, le brevet allemand 930 327, qui a trait à un procédé de préparation d'anhydrides d'acides carboxyliques par réaction du monoxyde de carbone (éventuellement en mélange avec de l'hydrogène) sur un ester (ou éther) méthylique dans la N-Méthylpyrrolidone en présence d'halogénure de nickel (ou de cobalt) ou d'autres composés de ces métaux (voire ces métaux eux-mêmes) en combinaison avec des halogènes libres ou combinés, insiste sur la nécessité d'opérer sous une pression d'au moins 200 atm.

Il a maintenant été trouvé qu'il est possible de préparer des anhydrides d'acides carboxyliques, en particulier des anhydrides d'acides alcanoïques inférieurs et notamment l'anhydride acétique par carbonylation d'esters carboxyliques en présence de nickel, et d'au moins un promoteur iodé, avec une bonne productivité, dans des conditions de pression relativement douces.

La présente invention a donc pour objet un procédé perfectionné de carbonylation d'esters carboxyliques de formule $R-CO-OR$ dans laquelle R représente un radical alkyl ayant au maximum 12 atomes de carbone ou un radical $C_6H_5-C_xH_{2x}-$, x étant un entier compris entre 1 et 6, inclus, én phase liquide comprenant un solvant choisi parmi les amides d'acides carboxyliques, en milieu sensiblement anhydre en présence d'une quantité efficace, de nickel et d'un promoteur halogèné, caractérisé en ce qu'on opère en présence d'au moins un cocatalyseur choisi parmi les composés ioniques des métaux alcalins et alcalino-terreux.

Le procédé selon la présente invention peut être représenté par le schéma réactionnel ci-après:

$$R-CO-OR + CO \rightarrow (RCO)_2O \qquad (1)$$

dans lequel R a la signification précédente.

R est avantageusement un radical alkyle ayant de 1 à 4 atomes de carbone tel que les radicaux méthyle, éthyle, n-propyle, isopropyle, n-butyle, butyle secondaire ou tertiobutyle.

Par milieu sensiblement anhydre, la Demanderesse entend un milieu ne renfermant que des traces d'eau susceptibles de provenir des réactifs et/ou des composants du système catalytique lorsqu'on souhaite faire usage de produits disponibles dans le commerce.

Le procédé selon l'invention nécessite la présence d'une quantité efficace de nickel. N'importe quelle source de nickel peut être mise en œuvre dans le cadre du présent procédé. On peut introduire le nickel sous sa forme métallique (nickel RANEY, par exemple) ou sous toute autre forme commode. A titre d'exemples de composés du nickel susceptibles d'être utilisés pour la mise en œuvre du présent procédé, on peut citer: le carbonate, l'oxyde, l'hydroxyde, les halogénures, en particulier l'iodure, les carboxylates, en particulier l'acétate, de nickel. Le nickel carbonyle convient également bien. On utilise de préférence le nickel RANEY, l'iodure de nickel, l'acétate de nickel et le nickel carbonyle.

La quantité de nickel n'est pas critique. La teneur en nickel qui a une influence sur la vitesse de réaction est déterminée en fonction de la vitesse de réaction que l'on estime convenable, compte tenu des autres paramètres de la réaction. De manière générale, une quantité comprise entre 5 et 2000 milliatomes-grammes de nickel par litre de solution conduit à des résultats satisfaisants. On opère de préférence avec une teneur comprise entre 20 et 1000 milliatomes-grammes de nickel par litre.

La mise en œuvre de la présente invention nécessite également la présence dans le milieu réactionnel d'au moins un iodure d'alkyle (ou d'acyle), c'est-à-dire d'au moins un composé de formule R'I (ou R'COI) dans laquelle R' a la signification donnée précédemment pour R, R' et R pouvant être identiques ou différents. On préfère utiliser les iodures d'alkyles ayant au maximum 4 atomes de carbone et plus particulièrement l'iodure de méthyle ou d'éthyle.

Il n'est pas nécessaire de charger initialement ce type de composant du système catalytique et l'on peut par exemple charger initialement de l'iode libre ou de l'acide iodhydrique.

La Demanderesse a constaté que, dans le milieu réactionnel, les iodures alcalins (ou alcalino-terreux) peuvent être considérés comme des précurseurs d'iodures d'alkyles. Les iodures de lithium, de sodium et de potassium conviennent bien à la mise en œuvre du présent prodédé. L'iodure de lithium

s'avère particulièrement efficace.

En général la quantité d'iodure d'alkyle (ou d'alkyle) ou de leur(s) précurseur(s) est telle que le rapport molaire I/Ni soit compris entre 3 et 50. Ce rapport est avantageusement fixé à une valeur comprise entre environ 5 et environ 30.

Le procédé selon l'invention nécessite en outre la présence d'un solvant choisi parmi les amides d'acides carboxyliques.

Par amides d'acides carboxyliques la Demanderesse entend les composés de formule:

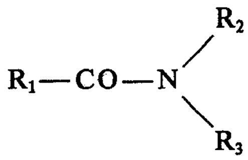

dans laquelle $R_1$, $R_2$ et $R_3$, identiques ou différents représentent un radical alkyle, un radical cycloalkyle, un radical aralkyle ou un radical aryle monocyclique ayant au plus 10 atomes de carbone, deux radicaux $R_1$, $R_2$ ou $R_3$ pouvant constituer ensemble un radical unique divalent $-(CH_2)_y-$, y étant un entier compris entre 3 et 12, $R_1$ pouvant en outre représenter un radical

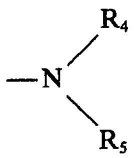

dans lequel $R_4$ et $R_5$, identiques ou différents représentent un radical alkyle ayant au maximum 4 atomes de carbone.

A titre d'exemple de tels solvants, on peut citer: la tétraméthylurée, le N,N-diméthylacétamide, le N,N-diéthylacétamide, le N,N-dicyclohexylacétamide, le N,N-diméthylpropionamide, le N,N-diéthylpropionamide, le N,N-diéthyl-n-butyramide, le N,N-diméthylbenzamide, le N,N-dicyclohexylbenzamide, le N,N-diéthyl-m-toluamide, la N-acétylpyrrolidine, la N-acétylpipéridine, la N-(n-butyryl)pipéridine, la N-méthylpyrrolidone-2, la N-éthylpyrrolidone-2, la N-méthylpipéridone-2, le N-méthyl-epsiloncaprolactame.

La N-méthylpyrrolidone-2 convient particulieèrement bien à la mise en œuvre du présent procédé.

En général la quantité de solvant représente au moins 10% en volume du milieu réactionnel; de bons résultats sont obtenus lorsqu'on en utilise de l'ordre de 20% à 75% en volume.

Une caractéristique essentielle du présent procédé réside dans la mise en œuvre d'au moins un cocatalyseur choisi parmi les composés ioniques des métaux alcalins et alcalino-terreux.

Les composés ioniques des métaux alcalins et alcalino-terreux utilisables dans le cadre du présent procédé répondent à la formule (II) ci-après:

$$M_b^{a+} X_c^{m-} \qquad \text{(II)}$$

dans laquelle a, m, b et c sont des entiers égaux à 1 ou à 2, dont les valeurs respectives sont choisies de sorte que la condition $a \times b = m \times c$ soit remplie et, lorsque a et m sont identiques, b et c sont égaux à 1; M représente un atome de lithium, de sodium, de potassium, de césium, de rubidium, de calcium ou de magnésium et $X^{m-}$ est un anion choisi dans le groupe constitué par: $OH^-$, $Cl^-$, $Br^-$, $I^-$, $CO_3^{=}$, $NO_3^-$, $R''-O^-$ et $R''-CO-O^-$, R″ ayant la signification donnée précédemment pour R, R″ et R pouvant être identiques ou différents.

Parmi ces composés, les composés ioniques des métaux alcalins et notamment les composés ioniques du lithium, du sodium ou du potassium conviennent particulieèrement bien à la mise en œuvre du présent procédé.

La nature précise de l'anion $X^{m-}$ ne paraît pas être un paramètre fondamental du présent procédé. A titre d'exemples de composés ioniques des métaux alcalins convenant à la mise en œuvre du présent procédé, on peut citer: LiOH, LiI, NaCl, KBr, NaI, KI, RbI, CsI, $NaNO_3$, $K_2CO_3$, $Li_2CO_3$, $CsNO_3$, l'acétate de lithium, de sodium ou de potassium, le méthylate de sodium ou de potassium et l'éthylate de sodium, de potassium ou de lithium. Les carboxylates alcalins ($R''-COOM$) et plus particulièrement les acétates sont d'un emploi commode et à ce titre peuvent être préconisés pour mettre en œuvre la présente invention.

A titre d'exemple de composés alcalino-terreux convenant à la mise en œuvre du présent procédé on peut citer: $Mg(OH)_2$, $MgI_2$, $Mg(OAc)_2$, CaI2 et $Ca(OAc)_2$. Les carboxylates et plus particulièrement les acétates alcalino-terreux sont d'un emploi commode et peuvent donc être préconisés pour mettre en œuvre la présente invention.

On constatera à la lecture du présent mémoire que les iodures alcalins ou alcalino-terreux dans le procédé selon l'invention peuvent être considérés non seulement comme cocatalyseurs, mais encore

comme précurseurs des iodures d'alkyles dont il a été fait mention précédemment. En d'autres termes, lorsqu'on introduit un iodure alcalin ou alcalino-terreux dans le milieu réactionnel il n'est pas nécessaire d'ajouter un iodure d'acyle (ou d'alkyle) et/ou un des cocatalyseurs définis ci-avant.

Bien entendu, dans le cadre du présent procédé, il est possible d'utiliser plusieurs cocatalyseurs. Ainsi, on peut mettre en œuvre un iodure alcalin et un carboxylate alcalin, par exemple l'iodure de sodium et l'acétate de lithium, ou l'iodure de lithium et l'acétate de potassium.

En général la présence de 0,5 à 50 moles de cocatalyseur par atome-gramme de nickel conduit à des résultats satisfaisants. Pour une bonne mise en œuvre du procédé selon l'invention on utilise de 1 à 25 moles de cocatalyseur par atome-gramme de nickel.

Selon la présente invention, on met en contact du monoxyde de carbone et un carboxylate d'alkyle en présence d'un solvant choisi parmi les amides d'acides carboxyliques et du système catalytique défini précédemment.

La réaction est conduite en phase liquide sous une pression supérieure à la pression atmosphérique. En général, on opère sous une pression totale supérieure à 15 bars; il n'est pas utile d'atteindre 700 bars. Pour une bonne mise en œuvre de l'invention, une pression totale de 25 à 200 bars est préconisée.

La température de réaction est généralement supérieure à 140° C, sans qu'il soit nécessaire d'atteindre 300° C. De bons résultats sont obtenus dans la gamme de température allant de 160 à 220° C.

On met en œuvre, de préférence, du monoxyde de carbone sous forme essentiellement pure, tel qu'il se présente dans le commerce. Toutefois, la présence d'impuretés telles que du dioxyde de carbone, de l'oxygène, du méthane et de l'azote peut être tolérée. La présence d'hydrogène n'est pas nuisible, même en des proportions relativement importantes.

En fin d'opération, l'anhydride carboxylique obtenu est séparé des autres constituants du milieu réactionnel par tout moyen approprié, par exemple par distillation.

Un avantage de la présente technique réside dans le fait que des compositions catalytiques particulièrement efficaces sont obtenues à partir d'espèces très accessibles et de structures extrêmement simples.

Une première catégorie de compositions catalytiques dont la mise en œuvre constitue une variante préférée de la présente invention est formée à partir de nickel et d'un iodure alcalin, en particulier un iodure de lithium, de sodium ou de potassium; l'iodure de lithium s'averrant particulièrement efficace.

Une seconde catégorie de compositions catalytiques dont l'utilisation constitue une autre variante avantageuse du présent procédé est formée à partir de nickel, d'un iodure d'alkyle et d'un carboxylate alcalin. L'iodure d'alkyle est avantageusement l'iodure de méthyle; le carboxylate alcalin est plus particulièrement un acétate, l'acétate de lithium convenant particulièrement bien.

Une dernière catégorie de compositions catalytiques préférées est obtenue à partir de nickel, d'un iodure d'alkyle, d'un iodure alcalin et d'un carboxylate alcalin; l'iodure de méthyle, l'iodure de sodium et l'acétate de lithium forment avec le nickel une composition catalytique particulièrement efficace.

Le procédé selon l'invention trouve une application particulièrement intéressante dans la préparation de l'anhydride acétique à partir de l'acétate de méthyle, dans la N-méthylpyrrolidone-2.

Les exemples ci-après illustrent la présente invention sans toutefois en limiter le domaine ou l'esprit.

Les conventions suivantes sont utilisées ci-après:

- AcOMe désigne l'acetate de méthyle
- NMP désigne la N-méthylpyrrolidone-2
- V désigne la vitesse initiale de la réaction exprimée en moles de monoxyde de carbone absorbées par heure.
- RR (%) désigne le nombre de moles d'anhydride acétique produites pour 100 moles d'acétate de méthyle chargées.
- Pr désigne la productivité en anhydride acétique exprimée en grammes par heure et par litre de milieu réactionnel.

## Exemple 1

Dans un autoclave en Hastelloy B 2, on charge:

- 25 ml (308 mMol) d'acétate de méthyle
- 20 ml de NMP (201 mMol)
- 8 mAt · g de nickel sous forme d'acétate tétrahydraté
- 83 mMol d'iodure de méthyle et
- 20 mMol d'iodure de sodium.

Après fermeture de l'autoclave, on établit une pression de 40 bars de monoxyde de carbone. L'agitation par un système de va-et-vient est mise en route et l'autoclave est porté à 180° C, en 25 minutes environ, au moyen d'un four annulaire. La pression dans l'autoclave est alors de 55 bars environ. Elle est ensuite maintenue constante et égale à 70 bars, par des recharges de monoxyde de

carbone. La chute de pression de la réserve haute pression qui alimente en continu l'autoclave est enregistrée. Après deux heures de réaction à la température indiquée, l'agitation et le chauffage sont arrêtés; l'autoclave est refroidi et dégazé. Le mélange réactionnel résultant est analysé.

Les résultats obtenus sont les suivants:

V = 0,20
RR = 22%
Pr = 65 g/h × l

Essai témoin a

On reproduit l'exemple 1 en l'absence d'iodure de sodium.

Les résultats obtenus sont les suivants:

V = 0,07
RR = 1,5%
Pr = 5 g/h × l

Exemples 2 à 5

Dans l'appareillage et selon le mode opératoire décrits ci-avant on réalise une série d'essais sur une charge constituée par de l'acétate de méthyle, de la NMP, 100 mMol d'iodure de sodium et 8 mAt-g de nickel sous forme d'acétate tétrahydraté. Les conditions particulières ainsi que les résultats obtenus en deux heures de réaction à 180°C sous une pression totale de 90 bars figurent dans le tableau ci-après.

| N° Ex | AcOMe ml | NMP ml | V | RR (%) | Pr |
|---|---|---|---|---|---|
| 2 | 45 | 5 | 0,02 | 1,8 | 10 |
| 3 | 40 | 10 | 0,035 | 7,8 | 40 |
| 4 | 30 | 20 | 0,14 | 35 | 130 |
| 5 | 20 | 30 | 0,14 | 38 | 95 |

Exemple 6

Dans l'appareillage et selon le mode opératoire décrits ci-avant on fait réagir du monoxyde de carbone sur une charge constituée par:

- 25 ml d'AcOMe (309 mMol)
- 22,5 ml de NMP
- 45 mMol d'iodure de méthyle
- 40 mMol d'iodure de sodium et
- 10 mAt-g de nickel sous forme d'acétate tétrahydraté.

Après deux heures de réaction à 180°C, sous une pression totale maintenue à 70 bars par des recharges de monoxyde de carbone on dose 13,7 g d'anhydride acétique (V=0,27; RR=48%, Pr=140 g/h × l).

Exemple 7

On reproduit l'exemple 5 ci-avant, mais en établissant après fermeture de l'autoklave, une pression de 101 bars de monoxyde de carbone. L'autoclave est alors porté à 180°C; la pression étant maintenue vers 150 bars par admission continue de monoxyde de carbone.

Les résultats obtenus en deux heures de réaction à 180°C sont les suivants:

V = 0,15
RR = 59%
Pr = 150 g/h × l

## Exemple 8

On reproduit l'exemple 4 en remplaçant dans la charge, l'iodure de sodium par la même quantité molaire d'iodure de lithium.

Les résultats sont les suivants:

V = 0,40
RR = 54%
Pr = 205 g/h × l

## Exemple 9

Dans l'appareillage et selon le mode opératoire décrits précédemment on fait réagir du monoxyde de carbone sur une charge constituée de:

- 25 ml d'AcOMe
- 20 ml de N,N-diméthylacétamide
- 80 mMol d'iodure de méthyle
- 23 mMol d'acétate de magnésium tétrahydraté et de
- 8 mAt-g de nickel sous forme d'acétate tétrahydraté.

Les résultats obtenus en deux heures de réaction à 180° C sous une pression totale de 70 bars sont les suivants:

V = 0,50
RR = 60%
Pr = 195 g/h × l

## Exemple 10

On reproduit l'exemple 1 en ajoutant à la charge 40 mMol d'acétate de lithium.

Les résultats obtenus sont les suivants:

V = 0,45
RR = 70%
Pr = 220 g/h × l

## Exemple 11

Dans un autoclave en Hastelloy B 2 et de 250 $cm^3$ de capacité, on charge:

- 25 ml (310 mMol) d'acétate de méthyle
- 70 ml de N-méthylpyrrolidone-2
- 81 mMol d'iodure de méthyle
- 100 mMol d'iodure de potassium et
- 20 mMol de nickel tétracarbonyle.

Après fermeture de l'autoclave, on admet une pression de 40 bars de monoxyde de carbone et on porte l'autoclave à 180° C, l'agitation étant assurée par un système de va-et-vient classique. La pression totale atteint alors 60 bars. On maintient cette pression pendant deux heures par admission continue d'oxyde de carbone à partir d'une réserve.

Les résultats, obtenus en deux heures de réaction à la température indiquée sont les suivants:

Pr = 110 g/h × l
RR = 68%

**Revendications**

1. Procédé de préparation d'anhydrides d'acides carboxyliques par carbonylation d'esters carboxyliques de formule RCOOR dans laquelle R représente un radical alkyle ayant au maximum 12 atomes de carbone ou un radical $C_6H_5-C_xH_{2x}-$, x étant un entier compris entre 1 et 6 inclus, en phase liquide comprenant un solvant choisi parmi les amides d'acides carboxyliques, en milieu sensiblement anhydre, en présence d'une quantité efficace de nickel et d'un promoteur halogéné, caractérisé en ce que la réaction est conduite en présence d'au moins un iodure d'alkyle ou d'acyle et d'au moins un cocatalyseur choisi parmi les composés ioniques des métaux alcalins et alcalino-terreux.

2. Procédé selon la revendication 1, caractérisé en ce que R est un radical alkyle ayant de 1 à 4 atomes de carbone.

3. Procédé selon la revendication 2, caractérisé en ce que R est un radical méthyle.

4. Procédé selon l'une quelconque des revendications précédentes, caractérisé en ce que les amides d'acides carboxyliques ont pour formule:

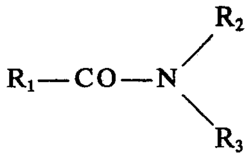

dans laquelle $R_1$, $R_2$ et $R_3$, identiques ou différents représentent un radical alkyle, un radical cycloalkyle, un radical aralkyle ou un radical aryle monocyclique ayant au plus 10 atomes de carbone, deux radicaux $R_1$, $R_2$ ou $R_3$ pouvant constituer ensemble un radical unique divalent $-(CH_2)_y-$, y étant un entier compris entre 3 et 12, $R_1$ pouvant en outre représenter un radical

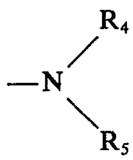

dans lequel $R_4$ et $R_5$, identiques ou différents représentent un radical alkyle ayant au maximum 4 atomes de carbone.

5. Procédé selon la revendication 4, caractérisé en ce que l'amide est la N-méthylpyrrolidone-2.

6. Procédé selon l'une quelconque des revendications précédentes, caractérisé en ce que l'amide représente au moins 10% en volume du milieu réactionnel.

7. Procédé selon la revendication 6, caractérisé en ce que l'amide représente de 20 à 75% en volume du milieu réactionnel.

8. Procédé selon l'une quelconque des revendications précédentes, caractérisé en ce que le cocatalyseur est un composé ionique d'un métal alcalin ou alcalino-terreux de formule:

$$M_b^{a+} X_c^{m-}$$

dans laquelle a, m, b et c sont des entiers égaux à 1 ou à 2, dont les valeurs respectives sont choisies de sorte que la condition $a \times b = m \times c$ soit remplie et, lorsque a et m sont identiques, b et c sont égaux à 1, M représente un atome de lithium, de sodium, de potassium, de césium, de rubidium, de calcium ou de magnésium et $X^{m-}$ est un anion choisi dans le groupe constitué par: $OH^-$, $Cl^-$, $Br^-$, $I^-$, $CO_3^=$, $NO_3^-$, $R''-O^-$ et $R''-CO-O^-$, R″ ayant la signification donnée précédemment pour R, R″ et R pouvant être identiques ou différents.

9. Procédé selon la revendication 8, caractérisé en ce que le cocatalyseur est un sel de lithium, de sodium ou de potassium.

10. Procédé selon la revendication 8 ou 9, caractérisé en ce que le cocatalyseur est un iodure.

11. Procédé selon la revendication 8 ou 9, caractérisé en ce que le cocatalyseur est un carboxylate.

12. Procédé selon la revendication 11, caractérisé en ce que le cocatalyseur est un acétate.

13. Procédé selon l'une quelconque des revendications précédentes, caractérisé en ce qu'on opère en présence d'un iodure d'alkyle ou d'acyle respectivement de formules R'I et R'COI dans lesquelles R' a la signification donnée précédemment pour R, R' et R pouvant être identiques ou différents.

14. Procédé selon la revendication 2 ou 3, caractérisé en ce qu'on opère en présence d'un iodure d'alkyle ayant au maximum 4 atomes de carbone, éventuellement formé in situ à partir d'un iodure alcalin ou alcalino-terreux.

15. Procédé selon l'une quelconque des revendications précédentes, caractérisé en ce qu'on opère en présence de 5 à 2000 et, de préference de 20 à 1000 milliatomes-grammes de nickel par litre de milieu réactionnel.

16. Procédé selon l'une quelconque des revendications précédentes, caractérisé en ce que le

rapport molaire I/Ni est compris entre 3 et 50 et, de préférence entre 5 et 30.

17. Procédé selon l'une quelconque des revendications précédentes, caractérisé en ce que le rapport molaire du cocatalyseur au nickel est compris entre 0,5 et 50 et, de préférence entre 1 et 25.

18. Procédé selon l'une quelconque des revendications précédentes, caractérisé en ce que la température est supérieure à 140° C et, est de préférence comprise entre 160 et 220° C.

19. Procédé selon l'une quelconque des revendications précédentes, caractérisé en ce que la pression totale est comprise entre 15 et 700 bars.

20. Procédé selon l'une quelconque des revendications précédentes, caractérisé en ce que la pression totale est comprise entre 25 et 200 bars.

**Patentansprüche**

1. Verfahren zur Herstellung von Carbonsäureanhydriden durch Carbonylieren von Carbonsäureestern der Formel RCOOR, in der R eine Alkylgruppe mit maximal 12 Kohlenstoffatomen oder einer Gruppe $C_6H_5-C_xH_{2x}-$, in der x eine ganze Zahl von 1 bis 6 ist, bedeutet, in flüssiger Phase umfassend ein Lösungsmittel ausgewählt unter den Carbonsäureamiden, in im wesentlichen wasserfreiem Medium, in Gegenwart einer wirksamen Menge Nickel sowie eines halogenhaltigen Promotors, dadurch gekennzeichnet, daß die Umsetzung in Gegenwart mindestens eines Alkyl- oder Acyljodids und mindestens eines Cokatalysators ausgewählt unter den ionischen Verbindungen von Alkali- und Erdalkalimetallen durchgeführt wird.

2. Verfahren nach Anspruch 1, dadurch gekennzeichnet, daß R eine Alkylgruppe mit 1 bis 4 Kohlenstoffatomen ist.

3. Verfahren nach Anspruch 2, dadurch gekennzeichnet, daß R eine Metylgruppe ist.

4. Verfahren nach einem der vorangehenden Ansprüche, dadurch gekennzeichnet, daß die Carbonsäureamide der Formel

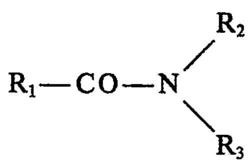

entsprechen, in der $R_1$, $R_2$ und $R_3$ gleich oder verschieden sind und (jeweils) für eine Alkylgruppe, eine Cycloalkylgruppe, eine Aralkylgruppe oder eine monozyklische Arylgruppe mit höchstens 10 Kohlenstoffatomen stehen, zwei Substituenten $R_1$, $R_2$ oder $R_3$ zusammen eine einzige zweiwertige Gruppe $-(CH_2)_y-$, in der y eine ganze Zahl von 3 bis 12 ist, bilden können und $R_1$ darüber hinaus eine Gruppe

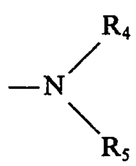

sein kann, in der $R_4$ und $R_5$ gleich oder verschieden sind und (jeweils) für eine Alkylgruppe mit maximal 4 Kohlenstoffatomen stehen.

5. Verfahren nach Anspruch 4, dadurch gekennzeichnet, daß das Amid N-Methylpyrrolidon-2 ist.

6. Verfahren nach einem der vorangehenden Ansprüche, dadurch gekennzeichnet, daß das Amid mindestens 10 Vol.-% des Reaktionsmediums ausmacht.

7. Verfahren nach Anspruch 6, dadurch gekennzeichnet, daß das Amid 20 bis 75 Vol.-% des Reaktionsmediums ausmacht.

8. Verfahren nach einem der vorangehenden Ansprüche, dadurch gekennzeichnet, daß der Cokatalysator eine ionische Verbindung eines Alkali- oder Erdalkalimetalls der Formel

$$M_b^{a+} X_c^{m-}$$

ist, in der a, m, b und c ganze Zahlen von 1 oder 2 sind, deren Werte so gewählt werden, daß die Bedingung $a \times b = m \times c$ erfüllt ist und, wenn a und m identisch sind, b und c jeweils 1 bedeuten, M für ein Lithium-, Natrium-, Kalium-, Caesium-, Rubidium-, Calcium- oder Magnesiumatom steht und $X^{m-}$ ein Anion ausgewählt aus der Gruppe $OH^-$, $Cl^-$, $Br^-$, $I^-$, $CO_3^{=}$, $NO_3^-$, $R''-O^-$ und $R''-CO-O^-$ ist, wobei R″ die zuvor für R angegebene Bedeutung hat und R″ und R gleich oder verschieden sein können.

9. Verfahren nach Anspruch 8, dadurch gekennzeichnet, daß der Cokatalysator ein Lithium-, Natrium- oder Kaliumsalz ist.

10. Verfahren nach Anspruch 8 oder 9, dadurch gekennzeichnet, daß der Cokatalysator ein Iodid ist.

11. Verfahren nach Anspruch 8 oder 9, dadurch gekennzeichnet, daß der Cokatalysator ein Carboxylat ist.

12. Verfahren nach Anspruch 11, dadurch gekennzeichnet, daß der Cokatalysator ein Acetat ist.

13. Verfahren nach einem der vorangehenden Ansprüche, dadurch gekennzeichnet, daß man in Gegenwart eines Alkyl- oder Acyliodids der Formel R'I bzw. R'COI arbeitet, wobei R' die zuvor für R angegebene Bedeutung hat und R' und R gleich oder verschieden sein können.

14. Verfahren nach Anspruch 2 oder 3, dadurch gekennzeichnet, daß man in Gegenwart eines Alkyliodids mit maximal 4 Kohlenstoffatomen arbeitet, das gegebenenfalls in situ ausgehend von einem Alkali- oder Erdalkaliiodid gebildet wird.

15. Verfahren nach einem der vorangehenden Ansprüche, dadurch gekennzeichnet, daß man in Gegenwart von 5 bis 2000, vorzugsweise von 20 bis 1000 mg-Atom Nickel je Liter Reaktionsmedium arbeitet.

16. Verfahren nach einem der vorangehenden Ansprüche, dadurch gekennzeichnet, daß das Molverhältnis I/Ni 3 bis 50, vorzugsweise 5 bis 30 ausmacht.

17. Verfahren nach einem der vorangehenden Ansprüche, dadurch gekennzeichnet, daß das Molverhältnis von Cokatalysator zu Nickel 0,5 bis 50, vorzugsweise 1 bis 25 ausmacht.

18. Verfahren nach einem der vorangehenden Ansprüche, dadurch gekennzeichnet, daß die Temperatur über 140° C, vorzugsweise im Bereich von 160 bis 220° C liegt.

19. Verfahren nach einem der vorangehenden Ansprüche, dadurch gekennzeichnet, daß der Gesamtdruck 15 bis 700 bar beträgt.

20. Verfahren nach einem der vorangehenden Ansprüche, dadurch gekennzeichnet, daß der Gesamtdruck 25 bis 200 bar ausmacht.

**Claims**

1. Process for the preparation of anhydrides of carboxylic acids by carbonylation of carboxylic esters of formula RCOOR in which R denotes an alkyl radical containing up to 12 carbon atoms or a radical $C_6H_5-C_xH_{2x}-$, x being an integer from 1 to 6 included, in a liquid phase containing a solvent chosen from the amides of carboxylic acids, in a substantially anhydrous medium, in the presence of an effective quantity of nickel and a halogen-based promoter, characterised in that the reaction is carried out in the presence of at least one alkyl iodide or acyl iodide and at least one co-catalyst chosen from the ionic compounds of the alkali metals and alkaline earth metals.

2. Process according to Claim 1, characterised in that R is an alkyl radical containing from 1 to 4 carbon atoms.

3. Process according to Claim 2, characterised in that R is a methyl radical.

4. Process according to any one of the preceding claims, characterised in that the amides of carboxylic acids have the formula:

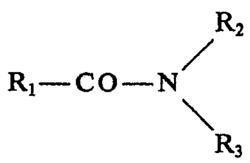

in which $R_1$, $R_2$ and $R_3$, which are identical or different, denote an alkyl radical, a cycloalkyl radical, an aralkyl radical or a monocyclic aryl radical containing up to 10 carbon atoms, two $R_1$, $R_2$ or $R_3$ radicals being capable of together forming a single divalent $-(CH_2)_y-$ radical, y being an integer from 3 to 12, $R_1$ being moreover capable of denoting a radical

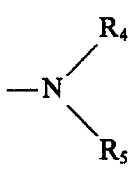

in which $R_4$ and $R_5$, which are identical or different, denote an alkyl radical containing up to 4 carbon atoms.

5. Process according to Claim 4, characterised in that the amide is N-methyl-2-pyrrolidone.

6. Process according to any one of the preceding claims, characterised in that the amide represents at least 10% by volume of the reaction medium.

7. Process according to Claim 6, characterised in that the amide represents from 20 to 75% by volume of the reaction medium.

8. Process according to any one of the preceding claims, characterised in that the co-catalyst is an ionic compound of an alkali metal or alkaline earth metal of the formula:

$M_b^{a+} X_c^{m-}$ (II)

in which a, m, b and c are integers equal to 1 or 2, the respective values of which are chosen so that the condition a × b = m × c is fulfilled and, when a and m are identical, b and c are equal to 1, M denotes a lithium, sodium, potassium, caesium, rubidium, calcium or magnesium atom and $X^{m-}$ is an anion chosen from the group consisting of: $OH^-$, $Cl^-$, $Br^-$, $I^-$, $CO_3^=$, $NO_3^-$, $R''-O^-$ and $R''-CO-O^-$, R'' having the meaning given earlier for R, R'' and R being capable of being identical or different.

9. Process according to Claim 8, characterised in that the co-catalyst is a salt of lithium, sodium or potassium.

10. Process according to Claim 8 or 9, characterised in that the co-catalyst is an iodide.

11. Process according to Claim 8 or 9, characterised in that the co-catalyst is a carboxylate.

12. Process according to Claim 11, characterised in that the co-catalyst is an acetate.

13. Process according to any one of the preceding claims, characterised in that it is carried out in the presence of an alkyl iodide or acyl iodide, having, respectively, the formulae R'I and R'COI, in which R' has the meaning given earlier for R, R' and R being capable of being identical or different.

14. Process according to Claim 2 or 3, characterised in that it is carried out in the presence of an alkyl iodide containing up to 4 carbon atoms, which is formed, if appropriate, in situ from an alkali metal iodide or an alkaline earth metal iodide.

15. Process according to any one of the preceding claims, characterised in that it is carried out in the presence of 5 to 2,000, and preferably from 20 to 1,000 milligramme-atoms of nickel per litre or reaction medium.

16. Process according to any one of the preceding claims, characterised in that the molar ratio I/Ni is between 3 and 50, and preferably between 5 and 30.

17. Process according to any one of the preceding claims, characterised in that the molar ratio of the co-catalyst to nickel is between 0.5 and 50, and preferably between 1 and 25.

18. Process according to any one of the preceding claims, characterised in that the temperature is above 140° C, and is preferably between 160 and 220° C.

19. Process according to any one of the preceding claims, characterised in that the total pressure is between 15 and 700 bars.

20. Process according to any one of the preceding claims, characterised in that the total pressure is between 25 and 200 bars.